# EUROPÄISCHE PATENTANMELDUNG

(11) **EP 0 694 512 A2**
(43) Veröffentlichungstag der Anmeldung: **31.01.1996**
(21) Anmeldenummer: 95106228.0
(22) Anmeldetag: 26.04.1995
(51) Int. Cl.: C07C 17/263, C07C 19/08

(54) **Verfahren zur Herstellung von Hexafluor-C4-Verbindungen**

(30) Priorität: 09.05.1994 DE 4416326
(71) Anmelder: BAYER AG, D-51368 Leverkusen (DE)
(72) Erfinder: Bielefeldt, Dietmar, Dr., D-40883 Ratingen (DE); Marhold, Albrecht, Dr., D-51373 Leverkusen (DE)

(57) **Zusammenfassung**

Hexafluor-C₄-Verbindungen werden aus Trifluorethanverbindungen in flüssiger oder gasförmiger Phase hergestellt, indem man diese mit einem Metall und/oder einer Metallverbindung in Kontakt bringt.

## Beschreibung

Die vorliegende Erfindung betrifft ein Verfahren zur Herstellung von Hexafluor-C4-Verbindungen durch dehalogenierende Dimerisierung von Trifluorethanverbindungen in flüssiger oder gasförmiger Phase.

Hexafluorbutane und Hexafluorbutene sind von Interesse als FCKW-Ersatzstoffe und als Zwischenprodukte zur Herstellung von Insektiziden und Akariziden (siehe z.B. DE-A 3 818 692 und EP-A 481 182).

Es ist bekannt, daß man Hexafluorchlorbutene herstellen kann, indem man chlorierte Butadiene einer Gasphasenfluorierung unterwirft (siehe DE-A-42 12 084). Dieses Verfahren erfordert die Handhabung von Fluorwasserstoff und Chlor in der Gasphase, was nur mit größerem technischen Aufwand möglich ist.

Hexafluorchlorbutene kann man auch durch Pyrolyse von 1,1,1-Trifluor-2,2-dichlorethan herstellen (siehe DE-A-42 14 739). Nachteilig hierbei ist, daß dieses Verfahren bei hohen Temperaturen durchgeführt werden muß. Der entstehende Chlorwasserstoff erfordert besonders korrosionsbeständige Materialien für den Pyrolysereaktor. Es ist also erheblicher technischer Aufwand nötig.

Man kann auch Verbindungen des Typs CF₃-CHal(Z)₂, wobei Hal für Chlor oder Brom und Z unabhängig davon für Wasserstoff, Chlor oder Brom steht, mit Wasserstoff in der Gasphase an Palladium und/oder Nickel enthaltenden Trägerkatalysatoren umsetzen und so Hexafluorbutan erhalten (siehe DE-A-42 15 876). Dabei ist nachteilig, daß die Standzeit des Katalysators, sowohl von seiner qualitativen und quantitativen Zusammensetzung, als auch von Z beeinflußt wird. Es ist also schwierig reproduzierbare Verhältnisse zu verifizieren, wenn man den Katalysator und/oder das Substrat ändert.

Es wurde nun ein Verfahren zur Herstellung von Hexafluor-C₄-Verbindungen der Formel (I)

CF₃-A-CF₃ (I),

in der
- A: für -CH₂-CH₂- oder -CX=CX- steht
und
- X: unabhängig voneinander jeweils für Wasserstoff, Chlor, Brom oder Iod steht,
gefunden, das dadurch gekennzeichnet ist, daß man eine Trifluorethanverbindung der Formel (II)

CF₃CXYZ (II)

in der
- X: die oben angegebene Bedeutung hat, und
- Y und Z: unabhängig voneinander für Wasserstoff, Fluor, Chlor, Brom oder Iod stehen,
jedoch mindestens einer der Substituenten von X, Y und Z von Wasserstoff verschieden ist
in flüssiger oder gasförmiger Phase mit einem Metall und/oder einer Metallverbindung in Kontakt bringt.

Beispiele für einsetzbare Verbindungen der Formel (II) sind 1,1,1-Trifluor-2,2,2-trichlorethan, 1,1,1-Trifluor-2-chlorethan, 1,1,1-Trifluor-2,2-dichlorethan, 1,1,1-Trifluor-2-brom-2-chlorethan, 1,1,1-Trifluor-2-jodethan, 1,1,1-Trifluor-2-bromethan und Gemische dieser Verbindungen.

Vorzugsweise werden 1,1,1-Trifluor-2,2,2-trichlorethan und 1,1,1-Trifluor-2,2-dichlorethan eingesetzt.

Das erfindungsgemäße Verfahren kann man in flüssiger Phase beispielsweise bei Temperaturen von -70 bis +200°C durchführen und in der Gasphase beispielsweise bei 100 bis 1200°C. Vorzugsweise arbeitet man in der flüssigen Phase bei 0 bis 200°C und in der Gasphase bei 400 bis 800°C.

Der Druck kann beispielsweise zwischen 0,1 mbar und 300 bar betragen, wobei man ihn so wählt, daß die gewünschte Phase vorliegt. Vorzugsweise arbeitet man in der flüssigen Phase bei Drucken von 100 mbar bis 100 bar und in der Gasphase bei Drucken von 0,8 bis 5 bar, insbesondere bei Normaldruck. Erhöhte Drucke kann man z.B. in Autoklaven realisieren, indem man darin das Reaktionsgemisch auf eine entsprechende Temperatur bringt und/oder Inertgase (z.B. Stickstoff oder Edelgase) aufdrückt.

In der Gasphase kann man gegebenenfalls in Gegenwart von Verdünnungsmitteln arbeiten, in der Flüssigphase gegebenenfalls in Gegenwart von Lösungsmitteln. Als Lösungsmittel kommen z.B. nicht-wäßrige polare Lösungsmittel in Frage wie Nitrile und Ether, insbesondere Acetonitril, Diglyme und Triglyme. Bezogen auf 1 g eingesetzter Verbindung der Formel (II) kann man beispielsweise 0,5 bis 20 ml Lösungsmittel einsetzen.

Als Metalle und Metallverbindungen kommen die verschiedensten in Frage. Bevorzugt sind Metalle und Verbindungen von Metallen der ersten Nebengruppe, der zweiten Haupt- und Nebengruppe und Eisen, Kobalt und Nickel. Die Metalle können z.B. einzeln, gemischt untereinander, gemischt mit anderen Metallen, als Legierungen untereinander oder als Legierungen mit anderen Metallen eingesetzt werden. Als Metallverbindungen kommen beispielsweise die Halogenide, insbesondere die Halogenide der niedrigen Oxidationsstufen der genannten Metalle in Frage. Die Metallverbindungen können z.B. als solche oder untereinander, mit anderen Metallverbindungen und/oder Metallen gemischt eingesetzt werden.

Die Menge an Metallen plus Metallverbindungen kann z.B. von 0,1 bis 10 g-Atom-Äquivalent, bezogen auf ein abzuspaltendes Halogenatom, betragen. Vorzugsweise liegt diese Menge zwischen 0,5 und 2 g-Atom-Äquivalent.

Es ist vorteilhaft, das Metall und/oder die Metallverbindung in pulvriger oder sonstiger feinverteilter Form einzusetzen. Das Metall und/oder die Metallverbindung kann auch auf einem Trägermaterial aufgebracht sein.

Man kann das erfindungsgemäße Verfahren gegebenenfalls unter der Einwirkung von Ultraschall durchführen, was insbesondere beim Arbeiten in flüssiger Phase und bei relativ niedrigen Drucken vorteilhaft ist.

Das erfindungsgemäße Verfahren ist diskontinuierlich und kontinuierlich durchführbar.

Die Aufarbeitung des nach der erfindungsgemäßen Umsetzung vorliegenden Reaktionsgemisches kann beispielsweise durch fraktionierte Destillation erfolgen. Wurde die erfindungsgemäße Umsetzung in der Gasphase durchgeführt, so ist es zweckmäßig, das gasförmige Reaktionsgemisch zu kondensieren.

Das erfindungsgemäße Verfahren hat die Vorteile, daß es ohne besonderen technischen Aufwand durchgeführt werden kann, sich auch bei wechselnden Katalysatoren und/oder Substraten relativ einfach reproduzierbare Verhältnisse verifizieren lassen und, bei Durchführung in der Gasphase, die Aufarbeitung sich einfach gestalten läßt.

### Beispiele

### Beispiel 1

In einem 0,7 l Emailleautoklav wurden bei 180°C 188 g CF₃CCl₃ mit 127 g Kupferpulver 15 Stunden bei einem maximal auftretenden Druck von 41 bar umgesetzt. Die aus dem Rückstand abdestillierten flüchtigen Reaktionsprodukte wurden mit Hilfe der gaschromatografischen gekoppelten Massenspektroskopie analysiert. Man erhielt ein Produktgemisch, das 83 Gew.-% CF₃-CCl=CCl-CF₃ (im folgenden DCHFB genannt) enthielt, sowie 6 Gew.-% nicht umgesetztes Edukt und 11 Gew.-% Chlorfluoralkane.

### Beispiel 2

In einem 0,7 l Autoklaven wurden bei 100°C 190 g CF₃CCl₃ mit 44 g Eisenpulver 4 Stunden in 150 ml Acetonitril als Lösungsmittel und in weiterer Gegenwart von 2 g FeCl₂ miteinander umgesetzt. Der maximal auftretende Druck betrug 7 bar. Die aus dem Rückstand abdestillierten flüchtigen Reaktionsprodukte wurden mit Hilfe der gaschromatografischen gekoppelten Massenspektroskopie analysiert. Das Produktgemisch enthielt 9 Gew.-% DCHFB und 90 Gew.-% nicht umgesetztes Edukt.

### Beispiel 3

In einen 3 l Autoklaven wurden zu 350 g Eisenpulver in 1000 ml Diglyme bei 180°C innerhalb von 10 Stunden 2345 g CF₃-CCl₃ zugepumpt. Das gebildete DCHFB wurde gleichzeitig durch eine Druckdestillation im Bereich von 10 bis 12 bar abdestilliert. Es wurden 287 g DCHFB mit einer gaschromatografisch ermittelten Reinheit von 92 % erhalten.

### Beispiel 4

In einem 0,3 l Autoklaven wurden bei 180°C 24 g CF₃CH₂Cl mit 6 g Eisenpulver 12 Stunden in 150 ml Diglyme als Lösungsmittel und in Gegenwart von 2 g FeCl₂ miteinander umgesetzt. Der maximal auftretende Druck betrug 7 bar. Die aus dem Rückstand abdestillierten flüchtigen Reaktionsprodukte enthielten 38 Gew.-% CF₃-CH₂-CH₂-CF₃.

### Beispiele 5 bis 8

Ein senkrecht stehendes beheizbares Quarzrohr wurde mit 200 ml Stahlwolle gefüllt. CF₃CCl₃ wurde über eine Dosierpumpe zugeführt. Nach Spülen mit Stickstoff wurde das CF₃CCl₃ in den angegebenen Mengen bei der jeweils angegebenen Temperatur durch das Quarzrohr geleitet. Das das Quarzrohr verlassende Reaktionsgemisch wurde bei -78°C kondensiert und mit Wasser versetzt, worauf sich eine organische und eine wäßrige Phase bildete. Die organische Phase wurde abgetrennt, über Natriumsulfat getrocknet und gaschromatografisch analysiert. Einzelheiten sind aus der Tabelle 1 ersichtlich.

**Tabelle 1**

| Beispiel Nr. | Temperatur (°C) | Belastung [g/l·h] | Umsatz (%) | Selektivität der Bildung von DCHFB (%) |
|---|---|---|---|---|
| 5 | 600 | 510 | 12,3 | 94 |
| 6 | 550 | 120 | 17,3 | 91 |
| 7 | 600 | 105 | 24,7 | 92 |
| 8 | 650 | 250 | 39,4 | 59 |

### Beispiele 9 bis 12

In einem Glaskolben wurden 79 g CF₃CCl₃ im Stickstoffstrom mit jeweils 20 g fein verteiltem Metall bei 38 bis 42°C im Ultraschallbad miteinander umgesetzt. Die Reaktionsdauer betrug jeweils 4 Stunden. Anschließend wurden die festen Bestandteile des Reaktionsgemisches abgefiltriert und die flüssige Phase gaschromatografisch untersucht. Einzelheiten sind aus der Tabelle 2 ersichtlich.

**Tabelle 2**

| Beispiel Nr. | Metall | Mol-Verhältnis CF₃CCl₃ : Metall | Selektivität der Bildung von DCHFB (%) |
|---|---|---|---|
| 9 | Fe | 1,17 | 97,1 |
| 10 | Zn | 1,37 | 96,6 |
| 11 | Mg | 0,51 | 97,3 |
| 12 | Cu | 1,33 | 84,1 |

## Patentansprüche

1. Verfahren zur Herstellung von Hexafluor-C₄-Verbindungen der Formel (I)
CF₃-A-CF₃ (I),
in der
A für -CH₂-CH₂- oder -CX=CX-
und
X unabhängig voneinander jeweils für Wasserstoff, Chlor, Brom oder Iod steht,
dadurch gekennzeichnet, daß man eine Trifluorethanverbindung der Formel (II)
CF₃CXYZ (II)
in der
X die oben angegebene Bedeutung hat,
Y und Z unabhängig voneinander für Wasserstoff, Fluor, Chlor, Brom oder Iod stehen,
jedoch mindestens einer der Substituenten X, Y und Z von Wasserstoff verschieden ist
in flüssiger oder gasförmiger Phase mit einem Metall und/oder einer Metallverbindung in Kontakt bringt.

2. Verfahren nach Anspruch 1, dadurch gekennzeichnet, daß man es in flüssiger Phase bei -70 bis +200°C oder in der Gasphase bei 100 bis 1200°C durchführt.

3. Verfahren nach Ansprüchen 1 und 2, dadurch gekennzeichnet, daß man in flüssiger Phase bei 100 mbar bis 100 bar oder in der Gasphase bei 0,8 bis 5 bar arbeitet.

4. Verfahren nach Ansprüchen 1 bis 3, dadurch gekennzeichnet, daß man in flüssiger Phase in Gegenwart von Lösungsmitteln arbeitet.

5. Verfahren nach Ansprüchen 1 bis 4, dadurch gekennzeichnet, daß man die Verbindung der Formel (II) mit Metallen und/oder Verbindungen von Metallen der ersten Nebengruppe, der zweiten Haupt- und/oder Nebengruppe und/oder Eisen, Kobalt und/oder Nickel in Kontakt bringt.

6. Verfahren nach Ansprüchen 1 bis 5, dadurch gekennzeichnet, daß man 0,1 bis 10 g-Atom-Äquivalent an Metallen und Metallverbindungen einsetzt, bezogen auf ein abzuspaltendes Halogenatom.
